# EUROPÄISCHE PATENTSCHRIFT

(11) **EP 0 627 623 B1**
(45) Veröffentlichungstag und Bekanntmachung des Hinweises auf die Patenterteilung: **08.08.2001**
(21) Anmeldenummer: 94104253.3
(22) Anmeldetag: 18.03.1994
(51) Int. Cl.: G01N 33/36

(54) **Verfahren und Vorrichtung zur Bestimmung der Struktur von Garnen im Bereich ihrer Oberfläche**
Method and apparatus for determining the surface structure of yarns
Méthode et appareil pour la détermination de la structure superficielle de fils

(30) Priorität: 31.03.1993 CH 98493; 02.04.1993 CH 102293
(43) Veröffentlichungstag der Anmeldung: 07.12.1994
(73) Patentinhaber: ZELLWEGER LUWA AG, 8610 Uster (CH)
(72) Erfinder: Hensel, Rolf, CH-8047 Zürich (CH); Wampfler, Hans, Dr., CH-8049 Zürich (CH); Seitz, Peter, Dr., CH-8700 Küsnacht (CH)
(74) Vertreter: Ellenberger, Maurice

(56) Entgegenhaltungen:
- WO-A-91/12490
- DE-A- 2 127 219
- FR-A- 1 584 684
- GB-A- 2 064 106
- US-A- 3 985 451
- US-A- 4 887 155

## Beschreibung

Es ist bekannt, dass es bei Rotorgarnen immer wieder vorkommt, dass Garne mit nahezu identischen Messwerten bei der Gleichmässigkeits- und Haarigkeistprüfung textile Flächengebilde mit stark unterschiedlichem Aussehen und voneinander abweichenden Eigenschaften (beispielsweise beim sogenannten "Griff") ergeben. Daraus kann man schliessen, dass gerade bei Rotorgarnen ein Mass oder Parameter zur textiltechnisch sinnvollen Charakterisierung der Garnoberflächen weitgehend fehlt. Man bedient sich heute als Hilfsmittel für die Strukturbestimmung eines Vergleichs zwischen der theoretisch eingestellten und der mechanisch gemessenen Drehung, wobei diese Messung durch ein Auf- und Zudrehverfahren erfolgt.

Dieses Auf- und Zudrehverfahren ist für Rotorgarne nicht nur ungenau und zeitaufwendig, sondern führt auch zu einer Zerstörung des zu untersuchenden Garns und genügt dadurch heutigen Anforderungen nicht mehr. Abgesehen davon ist speziell bei Rotorgarnen die Drehung, auch wenn sie exakt, rasch und zerstörungsfrei gemessen würde, kein ideales Mass für die Oberflächenstruktur, weil diese durch andere Phänomene wie beispielsweise die sogenannten Bauchbinden wesentlich stärker beeinflusst wird.

Bei Ringgarnen wird die Drehung ebenfalls mechanisch durch Auf- und Zudrehen gemessen, und bei Zwirnen sind ebenfalls mechanische Verfahren bekannt (siehe EP-A-118 466) = WO-A-84/00 781. Ausserdem sind noch optische Verfahren zur online Bestimmung von Drehungsschwankungen an Maschinen bekannt. Verfahren dieser Art sind beispielsweise in der CH-A-675 133, in der US-A-4,887,155 und in der DE-A-24 43 692 beschrieben.

Aus der WO 91/12490, welche den am nächsten kommenden Stand der Technik aufzeigt, ist ein Verfahren bekannt, gemäss welchem ein Lichtfleck auf das zu untersuchende Garn geworfen wird, worauf die Energieverteilung in diesem Lichtfleck analysiert wird um den Drehwinkel zu ermitteln. Der Lichtfleck ist kleiner als der Durchmesser des Garns. Die Richtung, in der die grösste Energie gemessen wird, wird als der gesuchte Drehwinkel genommen. Gemäss einer vorzugsweisen Ausführung wird auch die Richtung der grössten Ausdehnung des Lichtflecks gesucht und daraus der Drehwinkel bestimmt.

Ein Nachteil dieser bekannten Lösung ist darin zu sehen, dass Durchmesser und Drehwinkel eines Garns nicht aus einer einzigen Abbildung bestimmt werden kann. Hier braucht es einen ersten kleinen Lichtfleck um die Richtung der Fasern zu bestimmen und einen zweiten grösseren Lichtfleck um den Durchmesser des Garns zu bestimmen. Gemäss dieser Lösung ist zudem kohärentes einfarbiges Licht vorausgesetzt, so dass normales weisses oder farbiges Licht nicht verwendet werden kann. Hier stören auch abstehende Fasern und Muster im Lichtfleck die Messung.

Die vorliegende Erfindung betrifft ein Verfahren und eine Vorrichtung zur Bestimmung der Struktur von Garnen im Bereich ihrer Oberfläche. Das ist insbesondere die Struktur sowohl der eigentlichen Garnoberfläche als auch des Garnrandes einschliesslich von in diesem enthaltenen Einschlüssen oder von von diesem abstehenden Fasern. Dieses Verfahren soll genau, zerstörungsfrei und rasch arbeiten und somit auch zur online Messung an laufenden Maschinen eingesetzt werden können und es soll die Bestimmung von Parametern ermöglichen, die für eine textiltechnisch sinnvolle Charakterisierung der Garnoberflächen verwendet werden können.

Die Aufgabe wird erfindungsgemäss dadurch gelöst, dass das Licht auf mehrere Sensorelemente des Sensors geführt wird, wobei die Sensorelemente unterschiedliche Strukturierung aufweisen und die Strukturierungen Abbilder von typischen Arten der zu bestimmenden und im Licht enthaltenen Struktur darstellen, dass elektrische Signale aus diesen Sensorelementen verarbeitet und daraus die gesuchte Struktur bestimmt wird.

Unterschiedliche Strukturierung der Sensorelemente bedeutet in diesem Zusammenhang, dass die einzelnen Elemente beispielsweise verschieden orientiert oder maskiert sind. So können die Sensorelemente zur Drehungsmessung als Streifenmuster mit verschiedenem Neigungswinkel oder als verschieden geneigte schlitzförmige Fotodioden, und zur Bestimmung anderer Strukturmerkmale wie beispielsweise von Bauchbinden, als verschiedene Kopien typischer Bauchbinden ausgebildet sein.

Man kann bei der Bestimmung der gesuchten Struktur so vorgehen, dass man nur Sensoren für einen bestimmten Parameter verwendet; dann wird die Struktur anhand der Strukturierung der die beste Uebereinstimmung signalisierenden Sensorelemente bestimmt. Man kann aber auch mehrere Sensorarten für mehrere Parameter verwenden, und die Signale der Sensoren für die verschiedenen Parameter korrelieren, wobei dann auch eine sehr schlechte Uebereinstimmung für einen bestimmten Parameter signalisierenden Sensor unter Umständen einen Beitrag für die Bestimmung der gesuchten Struktur liefern kann.

Die erfindungsgemässe Vorrichtung ist dadurch gekennzeichnet, dass der Sensor aus einer Mehrzahl von fotoelektrischen Elementen mit unterschiedlicher Strukturierung aufgebaut ist, die typische Merkmale von Abbildungen enthalten, und dass eine Auswerteeinrichtung zur Verarbeitung der von den Sensorelementen gelieferten Signale vorgesehen ist, welche eine Korrelationsmatrix enthält.

Eine bevorzugte Ausführungsform der erfindungsgemässen Vorrichtung ist dadurch gekennzeichnet, dass die Sensorelemente durch auf einem integrierten Fotochip angeordnete fotoelektrische Elemente gebildet sind, und dass der Fotochip zusätzlich einen integrierten Schaltkreis für die Auswertung enthält.

Im folgenden wird die Erfindung anhand eines Ausführungsbeispiels und der Zeichnungen näher erläutert; es zeigt:
- Fig. 1: eine schematische Darstellung einer erfindungsgemässen Vorrichtung; und
- Fig. 2: einen stark vergrösserten Ausschnitt aus der Oberfläche des Sensors der Vorrichtung von Fig. 1.

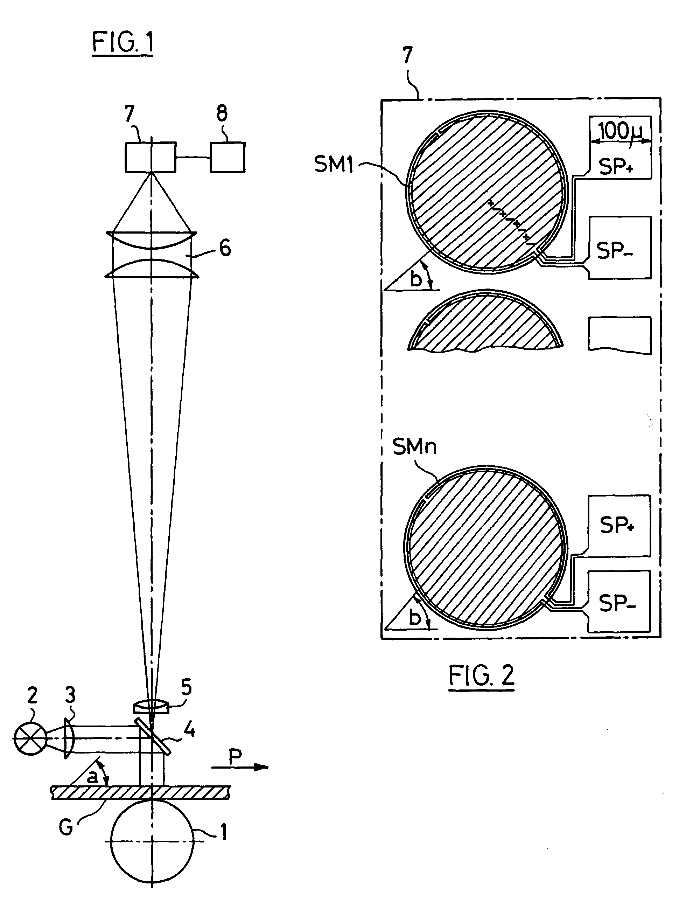

In Fig. 1 ist unten ein Stück eines Garns G dargestellt, welches durch nicht gezeigte Transportmittel in Richtung des Pfeiles P durch eine Vorrichtung zur Bestimmung der Oberflächenstruktur des Garns G gefördert wird. Ob die Förderung kontinuierlich und somit die Messung am bewegten Garn, oder ob die Förderung schrittweise und die Messung am stillstehenden Garn G erfolgt, ist für die Messung als solche von untergeordneter Bedeutung. In beiden Fällen ist das Garn G über eine Fadenführung 1 geführt, welche zur Positionierung des Garns im Strahlengang der Vorrichtung und zur Stabilisierung von dessen Lage dient.

Die einzelnen Windungen des Garns G weisen relativ zur Garnachse eine Steigung a auf. Bei Beleuchtung des Garns G senkrecht zu dessen Achse entstehen Reflexionen, die zur Bestimmung der Oberflächenstruktur des Garns auf einen geeigneten Sensor abgebildet werden. Wenngleich mit Oberflächenstruktur in erster Linie die Drehung des Garns gemeint ist, sind darunter auch kompliziertere Strukturen, wie beispielsweise Faserüberkreuzungen oder Bauchbinden zur verstehen, die beide ganz typische Strukturen aufweisen.

Das Garn G wird durch eine Lichtquelle 2 über einen Kondensor 3 und einen Strahlenteilerspiegel 4 senkrecht von oben beleuchtet, und das von der Garnoberfläche reflektierte Licht gelangt durch den Strahlenteilerspiegel 4 und ein Objektiv 5 zu einem Kondensor 6, der das Licht sammelt und auf einen fotoelektrischen Sensor 7 konzentriert. Dadurch entsteht auf dem Sensor 7 eine Abbildung der Oberflächenstruktur des Garns G die in einer mit dem Sensor 7 verbundenen Auswerteeinheit 8 näher bestimmt wird. Selbstverständlich kann das Garn G auch direkt beleuchtet werden.

Zur Ermöglichung der Bestimmung der Oberflächenstruktur des Garns G weist der Sensor 7 an seiner Oberfläche mehrere Fotoelementanordnungen mit unterschiedlicher Strukturierung auf, wobei die Strukturierungen Abbilder von typischen Arten der zu bestimmenden Struktur darstellen. Das Bild der Garnoberfläche wird mit diesen einzelnen Strukturtypen simultan oder sequentiell verglichen und es wird in einer Art von Korrelationsverfahren festgestellt, welche der Strukturtypen mit der zu bestimmenden Struktur am besten übereinstimmt.

Mit dem Begriff Struktur im Bereich der Oberfläche ist nicht nur die Struktur der eigentlichen Garnoberfläche gemeint, sondern auch diejenige des Garnrandes einschliesslich von Fasern, die von diesem abstehen, wobei man in diesem Fall die abstehenden Fasern innerhalb eines bestimmten Abstandes vom Garnrand berücksichtigen wird, und einschliesslich von eventuellen Einschlüssen. Zu den Einschlüssen sind auch Verunreinigungen zu zählen, insbesondere auch solche, die durch Fremdfasern verursacht sind. Verunreinigungen dieser Art können anhand ihrer Form und/oder anhand ihrer Farbe erkannt werden.

Im einfachsten Fall handelt es sich bei der Strukturbestimmung um die Messung des Faserwinkels a an der Garnoberfläche. Bei Ringgarnen kann aus dem Winkel a und dem Garndurchmesser die Drehung berechnet werden. Für diesen Anwendungsfall ist der Sensor 7 vorzugsweise gemäss der Darstellung von Fig. 2 ausgebildet, indem auf einem integrierten Fotochip mehrere, das sind in der Praxis bis zu 20 und mehr, Streifenmuster SM1 bis SMn von Fotoelementen angeordnet werden, die sich durch den Winkel b zur Horizontalen unterscheiden.

In Fig. 2 beträgt der Winkel b beim Streifenmuster SM1 40° und beim Streifenmuster SMn 50°; die Breite der einzelnen Streifen liegt in der Grössenordnung des Durchmessers der abgebildeten Fasern. Die in den einzelnen streifenförmigen Fotoelementen entstehenden Fotoströme werden abwechselnd auf einen von zwei Schaltungspunkten SP+ und SP- geführt und dort summiert, also die geradzahligen Fotoelemente auf den einen Schaltungspunkt und die ungeradzahligen auf den anderen SP+ bzw. SP-. In der Auswerteeinheit 8, die vorzugsweise durch einen auf dem Fotochip integrierten IC gebildet ist, wird die Differenz der Summenströme der beiden Schaltungspunkte bestimmt und ausgewertet. Diese über die Zeit gemittelte, absolute Differenz ist ein Mass für die Orientierung der Fasern und sie ist bei demjenigen Streifenmuster am grössten, dessen Winkel b mit dem Faserwinkel a an der Garnoberfläche am besten übereinstimmt. Der genaue Wert des Faserwinkels a wird durch Interpolation aus den Differenzen der Summenströme der einzelnen Streifenmuster erhalten.

Das beschriebene Verfahren, das einer Hochpassfilterung (Gradientenbildung) im Ortsraum entspricht, ist bewegungsunabhängig und funktioniert gleichermassen bei stehendem und bei bewegtem Garn G. Wenn man pro Winkel anstatt eines Streifenmusters nur ein einziges schlitzförmiges Fotoelement verwendet, dann ist die Messung bewegungsabhängig, indem bei bewegtem Garn für jedes Fotoelement ein zeitabhängiges Signal entsteht. Der von den Fasern stammende Signalanteil wird durch ein Hochpassfilter im Zeitbereich, vorzugsweise ein solches mit einer adaptiven Grenzfrequenz, herausgefiltert, wofür die ungefähre Garngeschwindigkeit bekannt sein muss. Und diese hochpassgefilterten Signalamplituden der einzelnen Fotoelemente werden dann linear interpoliert.

Aus dem mit einem der beiden beschriebenen Sensortypen ermittelten Faserwinkel a und aus dem Durchmesser des Garns G wird schliesslich die Garndrehung bestimmt. Die Messung des Durchmessers erfolgt vorzugsweise mit einer ebenfalls auf dem Fotochip enthaltenen Reihenzelle (Fotoelementzeile, CCD-Zeile, Fotodiodenzeile oder Transistorzeile). Anstatt eines einfachen Linienmusters können durch einen zusätzlichen absorbierenden Rasteraufdruck Fotoelementstrukturen mit beliebiger Empfindlichkeit erzeugt werden. Denn es ist möglich, ein beliebiges Ortsfilter theoretisch zu berechnen und dann im Ortsraum zu realisieren. So wird beispielsweise ein Rechteckfilter im Frequenzraum durch Fourier-Transformation ein sin(x)/x-Filter im Ortsraum. Die Bestimmung des Garndurchmessers ermöglicht es, den Sensor oder die Optik so zu regeln, dass diese immer auf die Garnmitte ausgerichtet sind.

Zur Erfassung und Bestimmung von komplizierteren Oberflächenstrukturen werden auf dem Fotochip Abbildungen von typischen Merkmalen der betreffenden Strukturen als Fotoelemente aufgebracht. Derartige Strukturen können beispielsweise Fotoelemente in Form eines Andreaskreuzes für Faserüberkreuzungen sein. Bei der Messung läuft die Abbildung der Garnoberfläche über die Fotoelementanordnung. Sobald das Garn typische Strukturmerkmale in der Art der Fotoelementanordnung enthält und diese auf den Sensor abgebildet werden, wird in den betreffenden Fotoempfängern der gleichen Struktur ein deutlicher Signalausschlag erfolgen, wogegen Empfänger mit unähnlichen Strukturen keinen Signalausschlag erzeugen. Dabei entspricht die Grösse des erzeugten Signals dem Produkt Kreuzkorrelation mal Lichtkontrast zwischen den beiden Strukturen. Durch die simultane Auswertung der Korrelationssignale von mehreren derartigen strukturierten Empfängern können die Garnstrukturen klassiert werden.

Durch Verwendung von einer oder mehreren Fotoelementzeilen eröffnet sich die zusätzliche Möglichkeit der Untersuchung des Garnrandes, was für die Erkennung von Bauchbinden hilfreich sein kann. Denn diese bilden einen welligen Garnrand und liegen satt am Garnkörper an, wodurch sie sich von abstehenden Fasern unterscheiden. Zusätzlich kann mit dieser Fotoelementzeile die Garnposition bestimmt, und es kann bei abnormal grossen Schwankungen des Garndurchmessers und/oder der Garnposition entschieden werden, ob das untersuchte Garn im gültigen Messbereich liegt.

Da der Garnrand im Unterschied zu der am besten im Auflicht zu sehenden Garnoberfläche besonder gut im Durchlicht zu sehen ist, empfiehlt sich die Anwendung von mindestens zwei verschiedenen Beleuchtungsarten oder Beleuchtungseinrichtungen. Diese können die Messungen von ein und derselben Stelle im Chopping-Betrieb oder an verschiedenen Stellen durchführen, wobei im letzteren Fall eine Synchronisierung der beiden Messarten erfolgen muss.

Zur Abbildung eines identischen Garnstücks auf verschiedene Sensoren können zeilen- oder matrixförmige Linsenanordnungen, sogenannte Linsenarrays, verwendet werden. Diese können auch für holografische Abbildungen ausgebildet sein. Die Auswertung der Hologramme der Oberflächenstrukturen könnte mit neuronalen Netzwerken erfolgen, mit denen verschiedene Strukturparameter untersucht und ausgewertet werden könnten. Diese verschiedenen Parameter spannen einen mehrdimensionalen Merkmalsraum auf, in dem eine bestimmte Struktur einen begrenzten Unterraum bildet.

Diffraktive optische Elemente und Linsenarrays zur Abbildung ein und desselben Garnstücks auf eine Vielzahl von Detektoren sind mit einem Laser als Beleuchtungsquelle gut zu realisieren, der bekanntlich mit monochromatischem und kohärentem Licht arbeitet. Denn bei Verwendung von monochromatischem Licht und einer Linse ergibt sich, dass die Fouriertransformation des Ortsbildes in der Brennebene der betreffenden Linse liegt. Aufgrund dieser Eigenschaft kann man optimale Filter zur Strukturierung auch in der Fourierebene definieren und als Fotoelementstruktur realisieren. Das hat den Vorteil, dass Garnposition und Tiefenschärfe keine grosse Rolle spielen, weil die Transformierte positionsunabhängig ist.

## Patentansprüche

1. Verfahren zur Bestimmung der Struktur von Garnen (G) im Bereiche ihrer Oberfläche, wobei das Garn beleuchtet und Licht vom Garn auf einen Sensor (7) geworfen wird, dadurch gekennzeichnet, dass das Licht auf mehrere Sensorelemente (SM1, SMn) des Sensors geführt wird, wobei die Sensorelemente unterschiedliche Strukturierung aufweisen und die Strukturierungen Abbilder von typischen Arten der zu bestimmenden und im Licht enthaltenen Struktur darstellen, dass elektrische Signale aus diesen Sensorelementen verarbeitet und daraus die gesuchte Struktur bestimmt wird.

2. Verfahren nach Anspruch 1, dadurch gekennzeichnet, dass die Strukturierungen der Sensorelemente Merkmale der Struktur eines Abbildes der Oberfläche des Garns aufweisen.

3. Verfahren nach Anspruch 1, dadurch gekennzeichnet, dass die Strukturierungen der Sensorelemente Merkmale der Fouriertransformation der Struktur der Oberfläche des Garns aufweisen.

4. Verfahren nach Anspruch 1 oder 2 zur Bestimmung der Garndrehung, dadurch gekennzeichnet, dass mit den genannten Sensorelementen (SM1, SMn) der Faserwinkel (a) an der Oberfläche und mit einem weiteren Detektor der Durchmesser des Garns (G) bestimmt und daraus die Garndrehung berechnet wird.

5. Verfahren nach Anspruch 1 oder 2, dadurch gekennzeichnet, dass zur Bestimmung komplizierter Strukturen auf dem Sensor (7) fotoelektrische Abbildungen von für diese Strukturen typischen Merkmalen hergestellt und die Abbildung des Garns (G) über diese Detektoranordnung geführt wird.

6. Verfahren nach Anspruch 5, dadurch gekennzeichnet, dass die zu bestimmenden Strukturen Garnverunreinigungen, insbesondere Verunreinigungen durch Fremdfasern sind.

7. Vorrichtung zur Durchführung des Verfahrens nach Anspruch 1, mit einer Lichtquelle (2) zur Beleuchtung eines Garns (G) und einem Sensor (7) zum Empfangen des Lichts vom Garn, dadurch gekennzeichnet, dass der Sensor (7) aus einer Mehrzahl von fotoelektrischen Elementen (SM1, SMn) mit unterschiedlicher Strukturierung aufgebaut ist, die typische Merkmale von Abbildungen enthalten, und dass eine Auswerteeinrichtung (8) zur Verarbeitung der von den Sensorelementen gelieferten Signale vorgesehen ist, welche eine Korrelationsmatrix enthält.

8. Vorrichtung nach Anspruch 7, dadurch gekennzeichnet, dass die Sensorelemente (SM1, SMn) durch auf einem integrierten Fotochip (7) angeordnete Fotoelemente gebildet sind, und dass der Fotochip zusätzlich integrierte Schaltkreise für die Auswertung enthält.

9. Vorrichtung nach Anspruch 8, dadurch gekennzeichnet, dass die fotoelektrischen Elemente (SM1, SMn) durch ein Streifenmuster von Fotoelementen gebildet und die Fotoströme der einzelnen Fotoelemente abwechselnd auf einen von zwei Schaltungspunkten (SP+, SP-) geführt sind, wo eine Summierung erfolgt, und dass die Differenzen der Summenströme der beiden Schaltungspunkte ein Mass für die Uebereinstimmung der Struktur des betreffenden Streifenmusters mit der untersuchten Struktur bildet.

10. Vorrichtung nach Anspruch 8, dadurch gekennzeichnet, dass auf dem integrierten Fotochip (7) eine zweite Art von Sensorelementen zur Bestimmung eines weiteren Garnmerkmals angeordnet ist.

11. Vorrichtung nach Anspruch 7, dadurch gekennzeichnet, dass die Anordnung zur Abbildung der Garnoberfläche ein sogenanntes Linsenarray zur Abbildung eines identischen Garn stücks auf mehrere Sensoren enthält.

## Claims

1. Method for determining the surface structure of yarns (G), the yarn being illuminated and light from the yarn being projected on to a sensor (7), characterized in that the light is directed on to several sensor elements (SM1, SMn) of the sensor, the sensor elements having different configurations and the configurations representing images of typical types of the structure to be determined and contained in the light, and in that electrical signals from these sensor elements are processed and the sought structure is determined from them.

2. Method according to Claim 1, characterized in that the configurations of the sensor elements comprise features of the structure of an image of the surface of the yarn.

3. Method according to Claim 1, characterized in that the configurations of the sensors elements comprise features of the Fourier transform of the structure of the surface of the yarn.

4. Method according to either of Claims 1 or 2 for determining the yarn twist, characterized in that the fibre angle (a) at the surface is determined by means of the said sensor elements (SM1, SMn) and the diameter of the yarn (G) is determined by means of a further detector, and the yarn twist is calculated from them.

5. Method according to either of Claims 1 or 2, characterized in that, for the purpose of determining complex structures, photoelectric images of features typical of these structures are produced on the sensor (7) and the image of the yarn (G) is directed over this detector arrangement.

6. Method according to Claim 5, characterized in that the structures to be determined are yarn impurities, particularly impurities due to foreign fibres.

7. Apparatus for execution of the method according to Claim 1, with a light source (2) for illuminating a yarn (G) and a sensor (7) for receiving the light from the yarn, characterized in that the sensor (7) is constructed from a plurality of photoelectric elements (SM1, SMn), of different configurations, which comprise typical features of images, and in that an evaluation device (8), comprising a correlation matrix, is provided for processing the signals delivered from the sensor elements.

8. Apparatus according to Claim 7, characterized in that the sensor elements (SM1, SMn) are formed by photoelements arranged on an integrated photochip (7) and in that the photochip additionally comprises integrated circuits for the evaluation.

9. Apparatus according to Claim 8, characterized in that the photoelectric elements (SM1, SMn) are formed by a striate pattern of photoelements and the photocurrents of the individual photoelements are directed alternately to one of two circuit points (SP+, SP-), where a summation is performed, and in that the differences of the summated currents of the two circuits points form a measurement of the match between the structure of the striate pattern concerned and the examined structure.

10. Apparatus according to Claim 8, characterized in that a second type of sensor element, for determining a second yarn feature, is disposed on the integrated photochip (7).

11. Apparatus according to Claim 7, characterized in that the arrangement for imaging the yarn surface comprises a so-called lens array for imaging an identical yarn portion on to several sensors.

## Revendications

1. Procédé pour la détermination de la structure de fils (G) au voisinage de leur surface, où le fil est éclairé et la lumière est projetée du fil sur un capteur (7), caractérisé en ce que la lumière est guidée sur plusieurs éléments (SM1, SMn) du capteur, où les éléments de capteur ont une structuration différente et les structurations sont des représentations de genres typiques de la structure à déterminer et contenue dans la lumière, en ce que des signaux électriques de ces éléments de capteur sont traités et que la structure recherchée est déterminée à partir de ceux-ci.

2. Procédé selon la revendication 1, caractérisé en ce que les structurations des éléments de capteur présentent des caractéristiques de la structure d'une représentation de la surface du fil.

3. Procédé selon la revendication 1, caractérisé en ce que les structurations des éléments de capteur présentent des caractéristiques de la transformation de Fourier de la structure de la surface du fil.

4. Procédé selon la revendication 1 ou 2, pour la détermination de la torsion du fil, caractérisé en ce qu'on calcule avec les éléments de capteur indiqués (SM1, SMn) l'angle de fibre (a) à la surface et avec un détecteur supplémentaire, le diamètre du fil (G) et à partir de ceux-ci la torsion du fil.

5. Procédé selon la revendication 1 ou 2, caractérisé en ce que, pour déterminer des structures compliqués sur le capteur (7), des représentations photoélectriques de caractéristiques typiques pour ces structures sont faites, et la représentation du fil (G) est guidée sur cet agencement de détecteurs.

6. Procédé selon la revendication 5, caractérisé en ce que les structures à déterminer sont des impuretés du fil, notamment des impuretés par des fibres étrangères.

7. Dispositif pour la mise en oeuvre du procédé selon la revendication 1, avec une source de lumière (2) pour éclairer un fil (G) et un capteur (7) pour recevoir la lumière du fil, caractérisé en ce que le capteur (7) est constitué d'une pluralité d'éléments photoélectriques (SM1, SMn) d'une structuration différente, qui contiennent des caractéristiques typiques de représentations, et en ce qu'il est prévu une installation d'évaluation (8) pour le traitement des signaux fournis par les éléments de capteur, qui contient une matrice de corrélation.

8. Dispositif selon la revendication 7, caractérisé en ce que les éléments de capteur (SM1, SMn) sont formés par des éléments photoélectriques disposés sur une photo-puce intégrée (7), et en ce que la photo-puce contient de plus des circuits intégrés pour l'évaluation.

9. Dispositif selon la revendication 8, caractérisé en ce que les éléments photoélectriques (SM1, SMn) sont formés par un dessin à rayures d'éléments photoélectriques, et que les courants photoélectriques des différents éléments photoélectriques sont guidés alternativement vers un de deux points de commutation (SP+, SP-), où a lieu une addition, et en ce que les différences des courants additionnés des deux points de commutation constituent une mesure pour la conformité de la structure du dessin à rayures concerné avec la structure examinée.

10. Dispositif selon la revendication 8, caractérisé en ce qu'il est disposé sur la photo-puce intégrée (7) un deuxième type d'éléments de capteur pour déterminer une autre caractéristique de fil.

11. Dispositif selon la revendication 7, caractérisé en ce que l'agencement pour la représentation de la surface du fil contient un soi-disant groupement de lentilles pour la représentation d'une pièce de fil identique sur plusieurs capteurs.
